Europäisches Patentamt

European Patent Office

Office européen des brevets

(11) Numéro de publication: **0 485 298 B1**

(12) **FASCICULE DE BREVET EUROPEEN**

(45) Date de publication de fascicule du brevet: **05.04.95**   (51) Int. Cl.6: **A01H 4/00**, A01H 1/00

(21) Numéro de dépôt: **91403012.7**

(22) Date de dépôt: **08.11.91**

(54) **Procédé de contrôle de la bulbification et/ou de la dormance lors de la micropropagation in vitro de plantes à bulbe.**

(30) Priorité: **08.11.90 FR 9013851**

(43) Date de publication de la demande:
**13.05.92 Bulletin 92/20**

(45) Mention de la délivrance du brevet:
**05.04.95 Bulletin 95/14**

(84) Etats contractants désignés:
**AT BE CH DE DK ES FR GB GR IT LI LU NL SE**

(56) Documents cités:
**EP-A- 0 150 528**
**LU-A- 33 071**

**AGRICULTURAL ENGINEERING vol. 44, no. 7, Juillet 1963,pages 368 - 369; & K.H.NORRIS: 'Effects of light on plants'**

**P.C.DEBERGH & R.H.ZIMMERMAN'Micropropagation,; Technology and application' 1991 , KLUWER ACADEMICPUBLISHERS , DORDRECHT & P.E.Read :chapter Micropropagation. pages 1-13**

**JOURNAL OF EXPERIMENTAL BOTANY vol. 31, no. 125, Décembre1980, pages 1675 - 1686; &A.FALAVIGNA: 'Origin and production**

of in vitro adventitious shoots in theonion,'

**Y.P.S.BAJAJ -ED- 'Biotechnology in agriculture and forestry 2; crops I.' 1985, SPRINGER VERLAG , BERLIN Section II, chapter II.7, pages 387-404;& J.DOLEZEL: "Onion, garlic and leek(Allium species)"**

(73) Titulaire: **COOP D'OR (SOCIETE COOPERATIVE)**
**Villers Les Pots**
**F-21130 Auxonne (FR)**

(72) Inventeur: **Kahane, Rémi**
**38 bis, rue de Tivoli**
**F-21000 Dijon (FR)**

(74) Mandataire: **Ores, Irène et al**
**CABINET ORES**
**6, Avenue de Messine**
**F-75008 Paris (FR)**

Rank Xerox (UK) Business Services
(3.10/3.09/3.3.3)

## Description

La présente Invention est relative à un procédé de contrôle de la bulbification et/ou de la dormance lors de l'obtention en vitroculture de plantes à bulbe et/ou de plantes appartenant à la famille des Liliacées.

Les techniques de vitroculture végétale sont couramment utilisées en agronomie. Une de leurs principales applications est la multiplication végétative *in vitro*, ou micropropagation, qui permet théoriquement l'obtention de milliers de plants identiques à la plante mère, à partir d'une seule plante. Cette technique a été utilisée avec succès pour la multiplication de nombreuses espèces végétales, à partir d'explants d'organes, de tissus ou de cellules.

Toutefois, s'il est en général relativement aisé de maintenir en culture n'importe quel fragment d'une plante, la régénération de plantes entières pose d'autres problèmes, car elle implique le contrôle de nombreux facteurs.

Différents procédés de culture des plantes à bulbes ont été proposés, à partir de différents types d'explants issus de la plante mère : culture de méristèmes, cultures de fragments d'organes, cultures de cals, cultures d'embryons.

De manière générale, les explants issus de la plante mère sont déposés sur un milieu de culture approprié ; au bout d'un certain temps, on assiste à l'apparition de bourgeons, puis de pousses herbacées groupées en touffes, et qui peuvent être repiquées individuellement ; il est possible d'obtenir à partir de la base des pousses individualisées de nouveaux explants qui peuvent théoriquement être mis en culture pour un nouveau cycle de multiplication.

De nombreuses recherches, ayant pour but la multiplication *in vitro* des plantes à bulbe ont été effectuées. A titre d'illustration, il est possible de se référer à une revue bibliographique, concernant la culture *in vitro* de l'ail, de l'oignon et du poireau, qui a été faite par NOVÄK et al. [Biotechnology in Agriculture and Forestry ; Vol 2 : Crops I ; Springer Verlag Eds, (1986)].

Il ressort des résultats des travaux réalisés jusqu'à présent, que s'il est possible, dans certains cas, d'obtenir *in vitro*, et au bout d'un cycle de multiplication, des plantules individualisées à partir de fragments d'une plante mère, de nombreux problèmes se posent pour effectuer des cycles de multiplication ultérieurs.

C'est ainsi, par exemple, que MATSUBARA et HIHARA [J. Jap. Soc. Hort. Sci., 46(4), 479-486, (1978)] ont réalisé un premier cycle de multiplication de l'oignon, mais n'ont pas controlé la bulbification observée, ni poursuivi la production de plantules *in vitro* au cours de cycles ultérieurs. FUJIEDA et al. [J. Jap. Soc. Hort. Sci., 48(2), 186-194, (1979)], sont également parvenus à effectuer un premier cycle de multiplication, mais n'ont pas réussi à produire une seconde génération de plantules à partir des pousses axillaires qu'ils ont obtenues.

Ultérieurement, HUSSEY [Scientia Hortic., 9, 227-236 (1978)], puis HUSSEY et FALAVIGNA [J. Exp. Bot. 31 : 125 1675-1686 (1980)] ont tenté d'effectuer plusieurs cycles de multiplication de l'oignon *in vitro* : ils ont ainsi constaté que les possibilités de cette technique étaient limitées par une baisse du coefficient de multiplication, et un arrêt de végétation de plus en plus précoce des plantules obtenues, au fil des générations. Ces résultats ont été confirmés, un peu plus tard, sur le narcisse [HUSSEY Ann. Bot. 49 701-719 (1982)].

Les phénomènes de blocage de végétation décrits par ces auteurs, bien que pouvant s'accompagner de la formation d'un "pseudo-bulbe" résultant du renflement de la base de la tige, ne sont pas similaires à ceux observés lors du processus de bulbification. Au cours de la bulbification, la formation d'un organe de réserve à la base de la plante s'accompagne en effet de la sénescence de l'appareil aérien, de l'arrêt du fonctionnement racinaire, et de l'entrée en dormance, c'est-à-dire de la non-reprise de végétation des organes bulbifiés, même cultivés dans des conditions favorables. Or, dans le processus décrit par HUSSEY et FALAVIGNA, il n'y a pas de sénescence des parties aériennes, pas de vieillissement des racines, et pas de dormance vraie.

Le déclenchement trop précoce d'un tel blocage de végétation doit être évité lors de la micropropagation *in vitro* des plantes à bulbe, car il oblige à une interruption de culture entre chaque cycle, ce qui diminue considérablement le rendement de la micropropagation *in vitro*, et par là même, l'intérêt de son utilisation.

Toutefois, en fin de culture, il conviendrait de procéder à l'induction d'une véritable bulbification pour obtenir des bulbes comparables aux bulbes cultivés *in vivo* et prêts à être repiqués en pleine terre.

Il serait donc particulièrement souhaitable de pouvoir contrôler, *in vitro*, le processus conduisant à la bulbification et à l'entrée en dormance afin de pouvoir soit le prévenir, soit au contraire l'induire au moment opportun.

Il est connu que la bulbification chez les plantes à bulbes cultivées en pleine terre est en rapport avec des variables telles que la durée et l'intensité de l'éclairement, et la température.

Les observations faites sur des plantes cultivées en pleine terre ne sont pas directement transposables *in vitro*, car il est pratiquement impossible de reproduire expérimentalement l'ensemble des variables complexes qui conditionnent le comportement des plantes cultivées en champ. En conséquence, le processus de bulbification *in vitro*, ainsi que les paramètres susceptibles de l'influencer ont fait l'objet de plusieurs études. Celles ci n'ont toutefois pas abouti à la mise en évidence du ou des facteurs essentiels dans le déclenchement de ce processus *in vitro*.

Une étude de HEATH et HOLLIES [J. Exp. Bot. 16(46), 128-144 (1965)] définit la bulbification par des critères morphologiques, qui sont essentiellement :

- le renflement de la base (quantifié par le rapport de bulbification, qui est égal au rapport du diamètre maximum à la base sur le diamètre au collet), et
- la présence d'écailles sur le bulbe formé.

Ces auteurs indiquent que la bulbification, telle qu'ils la définissent peut être induite, *in vitro*, par l'utilisation d'un milieu de culture riche en sucres.

Il a également été supposé que des substances telles que les auxines, les cytokinines, ou l'acide abscissique pouvaient favoriser la bulbification. Ces tentatives n'ont pas abouti, de façon satisfaisante, à l'obtention de bulbes comparables à ceux qui sont formés lors de cultures en pleine terre, à partir de semences.

Il a d'autre part été proposé, pour tenter cette fois de prévenir la bulbification en cultures *in vitro*, de soumettre lesdites cultures à des photopériodes courtes, d'environ 8 heures, ainsi que d'enrichir le milieu de culture en hormones de croissance, du type auxines, ou cytokinines [ BHOJWANI Scientia Hortic. 13 47-52 (1980)], [HUSSEY Scientia Hortic., 9, 227-236 (1978)], [HUSSEY et FALAVIGNA, J. Exp. Bot. 31(125), 1675-1686 (1980)], HUSSEY [Ann. Bot. 49 701-719 (1982)].

Il apparaît donc que jusqu'à présent, il n'a pas été possible de mettre en évidence le ou les facteurs induisant le processus de bulbification *in vitro*, et qu'aucun des procédés proposés pour contrôler ce processus, soit qu'il s'agisse de l'éviter pour améliorer le rendement de la micropropagation *in vitro*, soit qu'il s'agisse de le provoquer pour obtenir des bulbes viables, ne s'est montré réellement efficace.

La présente Invention s'est fixé pour but la mise au point d'une méthode de culture permettant le contrôle de l'entrée en dormance et/ou de la bulbification, au cours de la micropropagation *in vitro* des plantes à bulbe.

La micropropagation *in vitro* est constituée par une succession de cycles de multiplication. Un cycle de multiplication est défini comme la succession des trois étapes suivantes :

- une première étape, dite de régénération axillaire, dans laquelle des bourgeons sont régénérés à partir d'explants ;
- une deuxième étape, dite de croissance et développement en touffes herbacées, dans laquelle les bourgeons donnent naissance à des plantes qui se développent en touffes ;
- une troisième étape, dite d'individualisation, dans laquelle les touffes sont divisées en plantules qui sont cultivées individuellement jusqu'à enracinement et épaississement basal.

Au sens de la présente invention, on entend par bulbification le processus qui provoque chez un vitroplant entier :

- le renflement de la base, qui peut être évalué par le rapport de bulbification, défini plus haut ;
- la sénescence de l'appareil aérien ;
- l'arrêt du fonctionnement racinaire ;
- l'entrée en dormance, c'est à dire la non reprise de la végétation des organes bulbifiés cultivés en conditions favorables (environ 20°C, 12 à 16 heures de jour, et arrosages réguliers).

L'Inventeur est parvenu à agir sur les paramètres de culture, de façon à obtenir d'une part, la succession de cycles de multiplication, sans bulbification et sans entrée en dormance ni arrêt de végétation des plantules obtenues, et d'autre part, l'induction de la bulbification au moment souhaité ; la présente invention résulte en particulier de la constatation, faite par l'Inventeur, qu'un facteur essentiel du déclenchement de la bulbification *in vitro* est le rapport spectral rouge/rouge sombre de la source d'éclairage. L'éclairement des plantules, pendant 14 heures au moins par période de 24 heures, par une lumière contenant une proportion appropriée de radiations dans le rouge sombre suffit à induire le processus de bulbification, sans qu'il soit nécessaire d'agir sur les autres conditions de culture.

La présente Invention a pour objet un procédé permettant la micropropagation *in vitro* des plantes à bulbe et/ou des Liliacées par une succession de cycles de multiplication, dans lequel le contrôle de la dormance et/ou de la bulbification est effectué en modifiant le rapport entre l'énergie émise dans la région rouge (entre 600 et 700 nm), et l'énergie émise dans la région rouge sombre (entre 700 et 800 nm) par la source d'éclairage des cultures *in vitro*, lequel procédé est caractérisé en ce qu'il comprend au moins l'une des deux phases suivantes :

EP 0 485 298 B1

a) une phase de prévention de la bulbification, comprenant au moins un cycle de multiplication, dans lequel une photopériode comprenant entre 12 et 16 heures d'éclairement sur 24 heures est établie pendant la deuxième (croissance et développement en touffes herbacées) et la troisième étape (individualisation des plantules), et durant cette photopériode, l'éclairement des plantules est assuré par des sources lumineuses dont le rapport entre l'énergie émise, dans la région rouge et dans la région rouge sombre est supérieur ou égal à 4 ;

b) une phase d'induction de la bulbification, comprenant un cycle de multiplication dans lequel une photopériode d'au moins 14 heures et de préférence entre 16 heures et 18 heures d'éclairement sur 24 heures est établie pendant la deuxième et la troisième étape, et durant cette photopériode, l'éclairement des plantules est assuré par au moins une source lumineuse dont le rapport rouge/rouge sombre est inférieur ou égal à 4.

La première phase est mise en oeuvre dès le premier cycle et jusqu'à l'avant dernier cycle de multiplication ; cette phase permet de réaliser, sans bulbification et sans entrée en dormance, autant de cycles de multiplication qu'on le désire, pour obtenir le nombre de plantules souhaité ; à titre d'exemple, si l'on réalise de la sorte 3 cycles de multiplication, on obtient en un an 6000 à 8000 plantules à partir d'une plante mère. Lorsque l'on souhaite obtenir des bulbes, on réalise un dernier cycle de multiplication en mettant en oeuvre la phase d'induction de la bulbification.

Selon une disposition préférée de ce mode de mise en oeuvre, l'étape de régénération axillaire de chaque cycle de multiplication est effectuée en présence d'au moins une substance de croissance.

Des substances de croissance utiles pour obtenir une bonne régénération des bourgeons sont des cytokinines, par exemple la 6-benzyl-amino-purine utilisée à une concentration de 0,5 à 10 mg/l ; la kinétine, utilisée à une concentration de 1 à 20 mg/l, la 2-iso-pentenyl-adénosine, utilisé à une concentration de l'ordre de 1 à 20 mg/l. D'autres substances sont également bénéfiques à la régénération : il s'agit par exemple des auxines, telles que l'acide naphtalène-acétique, utilisé à une concentration de 0,1 mg/l, ou de la L-tyrosine, à une concentration de 10 à 100 mg/l. Des substances à activité comparable aux auxines et aux cytokinines peuvent également être utilisées ; il s'agit par exemple du piclorame (analogue des auxines) ou du dithiazuron (analogue des cytokinines) ; ces substances sont généralement utilisées à des concentrations de l'ordre de 0,1mg/ml.

D'autres paramètres du milieu de culture ont également une influence favorable sur l'étape de régénération axillaire des bourgeons :
- la concentration en sucres est de préférence comprise entre 30 et 50 g/l ;
- la concentration en gélifiant est comprise entre 5 et 10 g/l d'agar.

Les conditions de lumière et de température n'ont pas une influence déterminante sur l'étape de régénération axillaire ; la néoformation de bourgeons peut avoir lieu en jours continus ou à l'obscurité, et à des températures variant de $+5$ à $+35°C$.

Si on le souhaite, il est possible à la fin de l'étape de régénération axillaire d'un cycle de multiplication, de baisser la température du milieu de culture jusqu'à $+1°C$, ce qui a pour effet de stopper la croissance, et permet de conserver des plantules viables pendant au moins 6 mois sur le même milieu.

De manière avantageuse, au cours de la phase de prévention de la bulbification, la deuxième et la troisième étape du ou des cycle(s) de multiplication sont effectuées sur un milieu dépourvu de facteurs de croissance.

Préférentiellement, ledit milieu comprend au moins 20 meq de $NH_4^+$, au moins 20 meq de $NO_3^-$, au moins 3 meq de $PO_4^{3-}$ et entre 30 g/l et 60 g/l de carbohydrates ;

De manière avantageuse, au cours de la phase de prévention de la bulbification, la deuxième et la troisième étape du ou des cycle(s) de multiplication sont effectuées à une température diurne comprise entre $+20$ et $+30°C$ et supérieure d'au moins $+1°C$ à la température nocturne.

Comme indiqué ci-dessus, la bulbification est induite, conformément à l'Invention, par l'établissement d'une photopériode d'au moins 14 h d'éclairement sur 24 heures, à un rapport rouge/rouge sombre ≤ 4. La bulbification peut être en outre accélérée par des facteurs tels que l'augmentation de la teneur en sucres du milieu, la diminution des concentrations en sels minéraux, la présence d'acide abscissique, d'éthylène, ou d'autres substances de croissance, la culture à température élevée.

Avantageusement, au cours de la phase d'induction de la bulbification, la deuxième et la troisième étape du cycle de multiplication sont effectuées sur un milieu comprenant entre 30 et 80 g/l de sucres.

Si on le souhaite, il est également possible d'ajouter au milieu entre 0 et 5 mg/l d'acide abscissique, et/ou entre 0 et 1000 ppm d'éthylène.

L'éthylène est ajouté au milieu, par exemple sous forme d'acide 2-chloréthylphosphonique à des concentrations finales variant entre 0 et 10 mg/l

4

Toutefois, la composition du milieu n'intervient que pour accélérer le processus de bulbification et améliorer la qualité des bulbes. L'induction de la bulbification ne dépend que de la composition spectrale de la lumière d'éclairement, et de la durée de la photopériode.

Lorsque cela est souhaitable, au cours de la phase d'induction de la bulbification, la deuxième et la troisième étape du cycle de multiplication sont effectuées à une temperature diurne comprise entre +20 et +35°C et à une température nocturne comprise entre +15 et +30°C.

Une telle association de la thermopériode avec la photopériode est recommandée pour certaines espèces, comme l'oignon ; elle pourra être différente pour d'autres espèces. Pour chaque espèce, une association thermopériode-photopériode appropriée peut être déterminée à partir des conditions optimales de culture en champ, qui sont connues en elles-mêmes de l'homme de l'art.

Les milieux de base utilisables dans le cadre de la présente invention sont, de manière générale, tous les milieux habituellement utilisés pour la vitroculture de plantes, par exemple le milieu de MURASHIGE et SKOOG [Physiol. Plant. 15, 473-477, (1962)], le milieu de DUNSTAN et SHORT [Acta Hort., 78, 139-148 (1977)], ou des milieux dérivés de ceux-ci.

L'intensité de l'éclairement au niveau des plantes peut varier de 1000 à 10 000 lux, soit, en lumière blanche fluorescente, de 5 à 200 $\mu$E.m$^{-2}$.s$^{-1}$. L'éclairage peut comporter un apport de lumière blanche fluorescente du type "cool white" ou "warm white".

Les bulbes obtenus par le procédé conforme à l'invention sont réguliers, dormants et viables à 100% ; ils sont facilement acclimatables en pleine terre, et sont inductibles à fleurir dans l'année suivant leur transplantation en champ ; bien qu'hétérozygotes, ils se distinguent en outre des bulbes obtenus en pleine terre et issus de semences, par leur homogénéité génotypique, qui se traduit par une homogénéité phénotypique que ne présentent pas ces derniers.

Ils se distinguent d'autre part des pseudobulbes obtenus par des procédés connus dans l'art antérieur (milieux de culture à forte teneur en sucre et/ou en facteurs de croissance) par les caractéristiques suivantes :
- les bulbes obtenus conformément à l'invention présentent une période de dormance d'au moins un mois ; ils peuvent être facilement stockés et transportés, de la même manière que les bulbes obtenus en champ.
- L'induction de la bulbification par le procédé conforme à l'Invention qui fait intervenir des facteurs d'environnement, ne dépend pas de la composition du milieu de culture. Elle peut s'effectuer sur des milieux non enrichis en sucres (<60 g/l de saccharose par exemple). La teneur en sucres solubles des bulbes ainsi produits est donc nettement inférieure (d'au moins 20% à celle des pseudo-bulbes obtenus sur des milieux enrichis en sucres (>60 g/l de saccharose par exemple), ce qui se traduit également par une teneur en matière sèche plus faible (au moins 15%) des bulbes obtenus in vitro selon la méthode conforme à l'Invention.
- le rapport de bulbification des vitrobulbes obtenus conformément à l'invention est supérieur ou égal à 4(±0,6), alors qu'il est inférieur à cette valeur chez les pseudo-bulbes de l'art antérieur, obtenus sur des milieux enrichis en substances de croissance.

Le procédé de contrôle de la bulbification et de la dormance conforme à l'Invention est applicable à la micropropagation *in vitro* de toutes les plantes à bulbes, en particulier des Liliacées. Il est particulièrement bien adapté à la micropropagation des Alliacées à bulbe, comme l'oignon (*Allium cepa*), l'ail (*Allium sativum*), l'échalote (*Allium ascalonicum*).

En mettant en oeuvre le procédé conforme à l'Invention, l'induction de la bulbification peut se faire sans qu'il soit nécessaire de changer de milieu, ce qui présente un avantage inconstestable, tant du point de vue de l'abaissement du prix de revient, que de la simplification du protocole opératoire.

Il est bien entendu possible de mettre en oeuvre seulement l'une des deux phases du procédé conforme à l'invention. Par exemple, lorsque seules les conditions de prévention de la bulbification et/ou de la dormance sont mises en oeuvre, le procédé peut être utilisé dans la micropropagation d'espèces comme le poireau (*Allium porrum*), pour éviter l'entrée en dormance.

Ceci permet de nombreuses applications, parmi lesquelles il est possible de citer à titre d'exemple :
- la constitution d'une collection de clones à forte valeur génétique (mâles stériles, haploïdes ou polyploïdes, lignées pures d'allogames, etc...), les clones obtenus pouvant être soit conservés en culture sans dégénérescence, soit stockés sous forme de bulbes viables induits *in vitro*
- l'endomycorhization *in vitro* des espèces précitées, qui constitue une application particulièrement avantageuse : en effet, les endomycorhizes qui sont des champignons symbiotes des végétaux supérieurs ne peuvent se développer qu'au contact des cellules végétales vivantes du système racinaire, ce qui oblige à entretenir des plantes en serre spécialement pour conserver et multiplier lesdits champignons. L'entretien et la multiplication *in vitro* des Alliacées qui sont très mycotrophes à un stade de leur multiplication offre la possibilité de constituer *in vitro* un conservatoire actif de souches d'endomycorhizes. Ceci permet un gain d'espace, de main d'oeuvre, assure la pureté de la population d'endomycorhizes, et facilite l'échange de celles-ci directement sous forme de champignons.

La présente Invention sera mieux comprise à l'aide du complément de description qui va suivre, qui se réfère à des exemples de mise en oeuvre de procédés conformes à l'invention. Il va de soi toutefois que ces exemples sont donnés uniquement à titre d'illustration de l'objet de l'Invention dont ils ne constituent en aucune manière une limitation.

## I. CONDITIONS GENERALES DE CULTURE IN VITRO

### 1). PREPARATION DES EXPLANTS ET TECHNIQUES DE DESINFECTION

#### a) Sur inflorescence

Les organes floraux sont prélevés sur des plantes cultivées en plein champ, 12 heures au plus avant leur désinfection. Les hampes florales sont éliminées et les spathes fermées immergées dans l'éthanol 95% pendant une minute. Les inflorescences identifiées sont ensuite désinfectées dans une solution à 37g/l d'hypochlorite de calcium (70% de chlore actif) pendant 15 minutes puis rincées trois fois à l'eau stérile. Les explants tirés de l'inflorescence sont débarrassés de l'enveloppe de la spathe et placés face inférieure sur le milieu de culture.

Le milieu de culture se compose des éléments du milieu de MURASHIGE ET SKOOG additionnés des vitamines de MOREL ET MARTIN (cf Tableau I), de 20 g/l de saccharose, 7 g/l d'agar (BACTO) et d'hormones de croissance (0,5 g/l d'acide naphtalène acétique et 2,0 g/l de benzyl-amino-purine). Les cultures sont placées à 22-26°C sous 16 heures de photopériode (tubes fluorescents General Electric cool white 95 W) et $90\mu E.s^1.m^{-2}$.

#### b. Sur bulbe d'oignon

Les tissus prélevés se réduisent aux dix premières feuilles et ébauches foliaires de chaque apex enfermé dans un bulbe, chaque germe ainsi défini étant relié au plateau basal. Les germes à désinfecter sont préparés proprement, mais non stérilement en coupant le bulbe horizontalement en deux, en éliminant les écailles charnues communes à tous les germes, en découpant la base racinaire du bulbe, pour finalement ne conserver que les 3 premiers cm de chaque germe (écailles et feuilles) dont 0,5 cm de plateau basal, et moins de 2,5 cm de diamètre. On prend soin de laver chaque germe dans de l'eau tiède (50°C) savonneuse (Steepo) et de le rincer à l'eau avant de l'immerger 15 minutes dans l'éthanol à 95 % suivi par un bain de 40 minutes dans l'hypochlorite de calcium à 37 g/l (Prolabo).

En ambiance stérile, les germes sont rincés trois à quatre fois à l'eau distillée autoclavée. Les explants sont préparés en éliminant deux à trois écailles externes, ce qui réduit le diamètre et la taille des germes à 1 cm, et en découpant radialement au scalpel le germe en quatre ou six sections égales, détruisant par le fait l'apex central. Chaque section représente un explant, qui n'excède pas 0,5 $cm^3$ avec 8 segments de feuilles ou écailles maximum.

#### c. Sur vitroplant

Les plantes cultivées *in vitro* sont individualisées et l'on ne conserve d'elles que le plateau basal et 0,8 cm de feuilles au-dessus. Les racines en particulier seront coupées au niveau du plateau racinaire. Le "fût" de 0,8 cm de long mesure entre 0,5 et 1,5 cm de diamètre et comporte huit à dix bases foliaires. La préparation des explants consiste à couper le fût en deux suivant l'axe radial, puis de recouper radialement chaque moitié en deux parties égales ce qui achève de détruire le dôme apical.

### 2. MILIEUX DE CULTURE

La composition du milieu de culture de base (Ms) est la suivante : macro et micro-éléments de MURASHIGE ET SKOOG, vitamines de MOREL ET MARTIN [C.R.Ac.Agric.Fr., 41, 472-475, (1955)] diluées de moitié, agar bacto (DIFCO) (6.5 g/l). Quand elle n'est pas précisée, la teneur en sucre est de 40 g/l de saccharose très pur (Merck). Le pH est ajusté à 5,7 avec une solution normale de potasse ou d'acide chlorhydrique. Le milieu est autoclavé à 112 °C (1 bar) pendant 20 minutes.

Les substances de croissance et autres composants apportés au milieu de base ainsi défini sont incorporés avant d'ajuster le pH et autoclavés.

Les récipients de culture sont soit des tubes à essai (160x24 mm) en verre non hermétiquement clos contenant 13 ml de milieu, soit des bocaux en verre de 500 ml, remplis avec 100 à 150 ml de milieu et clos par un bouchon à vis.

## 3) CONDITIONS D'ENVIRONNEMENT

a). Salles climatisées

Trois salles peuvent héberger les vitro-cultures. Leurs caractéristiques sont indiquées dans le Tableau I suivant :

### TABLEAU I

| NUMERO DE SALLE | TEMPERATURES (et hygrométrie) | ECLAIREMENT |
|---|---|---|
| A | 15°C la nuit (50% HR)<br>25°C le jour (90% HR) | 12 heures de nuit<br>12 heures de jour<br>60 $\mu$E.s$^{-1}$.m$^{-2}$ |
| B | 22°C la nuit (50% HR)<br>25°C le jour (70% HR) | 8 heures de nuit<br>16 heures de jour<br>90 $\mu$E.s$^{-1}$.m$^{-2}$ |
| C | 20°C la nuit (50% HR)<br>20°C le jour (70% HR) | 12 heures de nuit<br>12 heures de jour<br>30 $\mu$E.s$^{-1}$.m$^{-2}$ |

La lumière blanche fluorescente est fournie par des tubes GENERAL ELECTRIC de type Cool White de 2 m de long et 95W de puissance nominative.

b). Enceintes climatisées

Ces enceintes sont prévues pour des températures allant de +5 à +30°C ±1.5°C. La thermopériode est couplée avec la photopériode, qui est un paramètre à définir. L'éclairage est fourni par trois tubes fluorescents Ostram Weiss White (Lumilux) de 40 cm de long et 15W de puissance nominative. A 25°C la quantité de lumière émise par trois tubes fluorescents est de 60 à 70 $\mu$E.s$^{-1}$.m$^{-2}$. L'hygrométrie doit être proche de 50% H.R. tout au long de l'année.

c) Chambre froide

La chambre froide est une pièce d'environ 100 m$^3$ climatisée et éclairée 12 heures sur 24. La température y varie de +1 à +5°C au niveau des récipients de culture. L'éclairage faible (10 $\mu$E.m$^{-2}$.s$^{-1}$) peut être amené à 70 $\mu$E.m$^{-2}$.s$^{-1}$ par deux tubes fluorescents General Electric de 95 W. L'hygrométrie y est inférieure à 40 % d'H.R.

d) Eclairement

La quantité de lumière active sur la photosynthèse (PAR) est mesurée par un radiophotomètre (LI-COR, Inc. modèle LI 185B avec cellule LI 190SB) au niveau des plantes mais à l'extérieur des récipients de culture ; elle s'exprime en $\mu$E.s$^{-1}$.m$^{-2}$

## II : CONTROLE DE LA BULBIFICATION

### 1. Introduction à partir d'inflorescence et de bulbe

Les milieux utilisés sont ceux de base (MS) avec 30 g/l de saccharose auxquels on apporte 0.2 mg/l d'acide naphtalène acétique et 2.0 mg/l de benzyl-amino-purine. Les explants sont cultivés dans les conditions de la salle B.

### 2. Etape de régénération et néoformation axillaire

Les substances de croissance ajoutées au milieu de base sont l'acide naphtalène acétique (0.20 mg/l à partir d'une solution aqueuse à 10$^{-4}$ g/l), la kinétine et la 2-iso-penténtyl-adénosine (2.5 à 5.0 mg/l à partir d'une solution aqueuse à 10$^{-4}$ g/l) et la benzyl-amino-purine (2.0 à 2.5 mg/l à partir d'une solutions aqueuse à 10$^{-4}$ g/l). Les conditions générales d'environnement sont celles de la salle B.

### 3. Etapes de croissance et développement herbacés, et d'individualisation des plantules

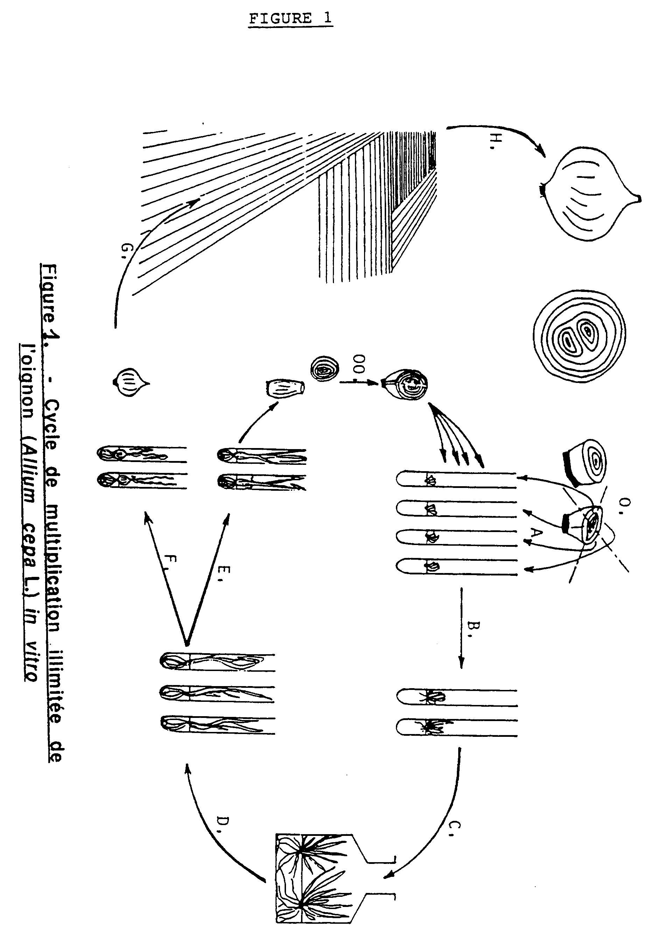

a) En conditions de prévention de la bulbification :
Ces étapes sont effectuées en l'absence de substances de croissance et le milieu MS de base est enrichi avec 300 mg/l d'orthophosphate de sodium et 40 mg/l d'adénine.
Les conditions de culture sont celles de la salle B; l'éclairage permet de maintenir un rapport rouge/rouge sombre égal à 6 ;
8 cycles successifs ont ainsi été effectués à partir d'un bulbe d'oignon ; au huitième cycle, à partir de 96 explants issus des plantules obtenues au 7ème cycle, 80 ont régénéré des bourgeons (816 bourgeons), soit un taux de régénération de 83%. A titre de comparaison, le bulbe initial avait donné 24 explants dont 11 avaient régénéré des bourgeons (43 bourgeons), soit un taux de régénération de 46%.
b) En conditions d'induction de la bulbification :
Le milieu de culture du vitroplant individualisé est le milieu MS de base, auquel est ajouté du saccharose, pour une concentration finale de 40 g/l.
Les conditions de culture sont les suivantes :
- Photopériode de 16 heures de jour ; l'éclairage est fourni par les sources lumineuses de la salle B enrichies en rouge sombre par l'adjonction d'ampoules à incandescence de 25 W, à raison d'une ampoule pour 1,5 m², ce qui permet de maintenir un rapport rouge/rouge sombre inférieur à 2 ;
- La température est maintenue à 22°C pendant la nuit, et 25°C pendant le jour.
Les étapes d'un cycle de multiplication de l'oignon dans le cadre du procédé conforme à l'Invention sont illustrées à la figure 1.
La légende de cette figure est la suivante :
O.     Préparation et désinfection des explants à partir d'un bulbe
A.     Mise en culture des explants primaires (I) et secondaires (II) sur milieu contenant des hormones
B.     Régénération des bourgeons sur milieu contenant auxine et cytokinine
C.     Croissance et développement des bourgeons régénérés en plantes entières individualisables, sur milieu sans hormones
D.     Individualisation des vitroplants sur milieu sans hormones
E.     Elargissement du plateau basal par une production de nouvelles feuilles sur milieu sans hormone et riche en sucre
F.     Bulbification des vitroplants par enrichissement de la lumière en rouge sombre en photopériode de 16 h, sur milieu sans hormones
G.     Plantation au champ
H.     Récolte des bulbes d'oignons
OO.     Préparation des explants à partir de vitroplantsmères.
Le tableau II ci-dessous montre la comparaison entre la teneur en sucres solubles d'un bulbe obtenu par le procédé conforme à l'Invention (3) avec celle d'un pseudo-bulbe obtenu sur milieu enrichi en sucre

8

(2) et avec celle de la base non bulbifiée d'une plantule (1).

TABLEAU II

| Condition de culture [a] | 1 | 2 | 3 |
|---|---|---|---|
| | 40 F | 80 F | 40 F+I |
| Teneur en sucres solubles [b] | 28.0 | 42.6 | 25.1 |

a) 40 ou 80 g/l de saccharose dans le milieu de culture, éclairage en photopériode de 16 heures avec lumière fluorescente uniquement (F) ou enrichie en incandescence (F+I) ;

b) Glucose + fructose + saccharose, en g/kg de matière fraîche.

La teneur en sucres solubles d'un bulbe conforme à l'Invention est inférieure de 41% à celle d'un pseudo-bulbe obtenu sur milieu enrichi en sucre.

### III. ACCLIMATATION EX-VITRO

Les vitrobulbes obtenus au bout d'un cycle de multiplication dans les conditions d'induction de la bulbification décrites ci-dessus sont séchés pendant une semaine à température ambiante, avant d'être placés sur un mélange tourbe/gravier arrosé tous les 3 jours.

La durée de la dormance varie selon les conditions climatiques ; elle est d'environ 65 jours en jours courts (inférieurs à 12 heures), et à une température de 20°C constante, ainsi qu'en jours longs, à température constante de 9°C, de 40 jours environ en jours longs, à température nocturne de 20°C et diurne de 25°C, et de 35 jours environ si les bulbes sont placés en jours longs, à température constante de 9°C pendant 20 jours, puis à température nocturne de 20°C et diurne de 25°C.

Ainsi que cela ressort de ce qui précède, l'Invention ne se limite nullement à ceux de ses modes de mise en oeuvre, de réalisation et d'application qui viennent d'être décrits de façon plus explicite.

### Revendications

1. Procédé permettant la micropropagation des plantes à bulbe et/ou des Liliacées par une succession de cycles de multiplication *in vitro*, chacun desdits cycles comprenant trois étapes à savoir: étape de régénération axillaire, étape de croissance et développement, étape d'individualisation, dans lequel le contrôle de la dormance et/ou de la bulbification des plantes cultivées *in vitro* est effectué en modifiant le rapport entre l'énergie émise dans la région rouge, et l'énergie émise dans la région rouge sombre par la source lumineuse assurant l'éclairage desdites plantes, lequel procédé est caractérisé en ce qu'il comprend au moins l'une des deux phases suivantes :

   a) une phase de prévention de la bulbification, comprenant au moins un cycle de multiplication, dans lequel une photopériode comprenant entre 12 et 16 heures d'éclairement sur 24 heures est établie pendant la deuxième et la troisième étape, et durant cette photopériode, l'éclairement des plantules est assuré par des sources lumineuses dont le rapport entre l'énergie émise, dans la région rouge et dans la région rouge sombre est supérieur ou égal à 4 ;

   b) une phase d'induction de la bulbification, comprenant un cycle de multiplication dans lequel une photopériode d'au moins 14 heures d'éclairement sur 24 heures est établie pendant la deuxième et la troisième étape, et durant cette photopériode, l'éclairement des plantules est assuré par au moins une source lumineuse dont le rapport rouge/rouge sombre est inférieur ou égal à 4.

2. Procédé selon la Revendication 1, caractérisé en ce que pendant la phase d'induction de la bulbification, le rapport rouge/rouge sombre de la source lumineuse assurant l'éclairement des plantules est inférieur ou égal à 2.

3. Procédé selon l'une quelconque des Revendications 1 ou 2, caractérisé en ce que au cours de la phase d'induction de la bulbification, une photopériode comprenant entre 16 et 18 heures d'éclairement est établie pendant la 2ème et la 3ème étape du cycle de multiplication.

4. Procédé selon l'une quelconque des Revendications 1 à 3, caractérisé en ce que la première étape de chaque cycle de multiplication est effectuée en présence d'au moins une substance de croissance.

5. Procédé selon une quelconque des revendications 1 à 4 caractérisé en ce que, au cours de la phase de prévention de la bulbification, la deuxième et la troisième étape du ou des cycle(s) de multiplication sont effectuées sur un milieu dépourvu de facteurs de croissance.

6. Procédé selon la Revendication 5, caractérisé en ce que le milieu comprend au moins 20 meq de $NH_4^+$, au moins 20 meq de $NO_3^-$, au moins 3 meq de $PO_4^{3-}$ et entre 30 g/l et 60 g/l de sucres ;

7. Procédé selon l'une quelconque des Revendications 1 à 6, caractérisé en ce que, au cours de la phase de prévention de la bulbification, la deuxième et la troisième étape du ou des cycle(s) de multiplication sont effectuées à une température diurne comprise entre +20 et +30°C et supérieure d'au moins +1°C à la température nocturne.

8. Procédé selon l'une quelconque des Revendications 1 à 7, caractérisé en ce que, au cours de la phase d'induction de la bulbification, la deuxième et la troisième étape du cycle de multiplication sont effectuées sur un milieu comprenant entre 30g/l et 80g/l de sucres

9. Procédé selon l'une quelconque des Revendications 1 à 8, caractérisé en ce que, au cours de la phase d'induction de la bulbification, la deuxième et la troisième étape du cycle de multiplication sont effectuées sur un milieu comprenant entre 0 et 5 mg/l d'acide abscissique, et/ou entre 0 et 1000 ppm d'éthylène.

10. Procédé selon l'une quelconque des Revendications 1 à 9, caractérisé en ce que, au cours de la phase d'induction de la bulbification, la deuxième et la troisième étape du cycle de multiplication sont effectuées à une température diurne comprise entre +20 et +35°C et à une température nocturne comprise entre +15 et +30°C.

11. Procédé, selon la revendication 1, caractérisé en ce qu'il comprend uniquement la phase de prévention de la bulbification.

12. Procédé, selon la revendication 1, caractérisé en ce qu'il comprend uniquement la phase d'induction de la bulbification.

**Claims**

1. Method for the micropropagation of bulb plants and/or Liliaceae by a succession of in vitro multiplication cycles, where each of said cycles comprises three steps, namely: axillary regrowth step, growth and development step and individualization step, in which method control of the dormancy and/or bulb formation of the plants grown in vitro is effected by changing the ratio between the energy emitted in the red spectral range and the energy emitted in the far-red spectral range by the light source which provides illumination of said plants, which method is characterized in that it comprises at least one of the two phases below:
   a) a bulb-formation prevention phase which comprises at least one multiplication cycle in which a photoperiod comprising between 12 and 16 hours of illumination per 24 hours is established during the second and third steps, during which photoperiod illumination of the plantlets is provided by light sources whose ratio between the energy emitted in the red spectral range and in the far-red spectral range is greater than or equal to 4;
   b) a bulb-formation induction phase which comprises a multiplication cycle in which a photoperiod of at least 14 hours illumination per 24 hours is established during the second and third steps, and during this photoperiod illumination of the plantlets is provided by at least one light source whose red/far red ratio is less than or equal to 4.

2. Method according to Claim 1, characterized in that during the bulb-formation induction phase the red/far red ratio of the light source which provides illumination of the plantlets is less than or equal to 2.

3. Method according to any one of Claims 1 or 2, characterized in that during the bulb-formation induction phase a photoperiod comprising between 16 and 18 hours of illumination is established during the 2nd and 3rd steps of the multiplication cycle.

4. Method according to any one of Claims 1 to 3, characterized in that the first step of each multiplication cycle is effected in the presence of at least one growth substance.

5. Method according to any one of Claims 1 to 4, characterized in that during the bulb-formation prevention phase, the second and third steps of the multiplication cycle(s) are effected on a medium lacking growth factors.

6. Method according to Claim 5, characterized in that the medium comprises at least 20 meq of $NH_4{}^+$, at least 20 meq of $NO_3{}^-$, at least 3 meq of $PO_4{}^{3-}$ and between 30 g/l and 60 g/l of sugars.

7. Method according to any one of Claims 1 to 6, characterized in that during the bulb-formation prevention phase, the second and third steps of the multiplication cycle(s) are effected at a daytime temperature of between $+20\,°C$ and $+30\,°C$ which is at least $+1\,°C$ above the nighttime temperature.

8. Method according to any one of Claims 1 to 7, characterized in that, during the bulb-formation induction phase, the second and third steps of the multiplication cycle are effected on a medium comprising between 30 g/l and 80 g/l of sugars.

9. Method according to any one of Claims 1 to 8, characterized in that, during the bulb-formation induction phase, the second and third steps of the multiplication cycle are effected on a medium comprising between 0 and 5 mg/l of abscisic acid and/or between 0 and 1,000 ppm of ethylene.

10. Method according to any one of Claims 1 to 9, characterized in that, during the bulb-formation induction phase, the second and third steps of the multiplication cycle are effected at a daytime temperature of between $+20$ and $+35\,°C$ and a nighttime temperature of between $+15$ and $+30\,°C$.

11. Method according to Claim 1, characterized in that it comprises solely the bulb-formation prevention phase.

12. Method according to Claim 1, characterized in that it comprises solely the bulb-formation induction phase.

**Patentansprüche**

1. Verfahren zur Mikrovermehrung von Zwiebelpflanzen und/oder Liliazeen durch eine Aufeinanderfolge von Vermehrungszyklen in vitro, wobei jeder der Zyklen drei Schritte umfaßt, nämlich einen Schritt der Regeneration der Achselknospen, einen Wachstums- und Eintwicklungsschritt, und einen Individualisierungsschritt, wobei die Kontrolle der Keimruhe und/oder der Zwiebelbildung der in vitro gezüchteten Pflanzen durch Modifizieren des Verhältnisses zwischen der von der Lichtquelle, die die Beleuchtung der Pflanzen gewährleistet, im roten Bereich ausgestrahlten Energie und der im dunkelroten Bereich ausgestrahlten Energie gewährleistet wird, dadurch **gekennzeichnet,** daß es mindestens eine der folgenden beiden Phasen umfaßt:
   (a) eine Phase der Verhinderung der Zwiebelbildung, umfassend mindestens einen Vermehrungszyklus, in dem eine Photoperiode von zwölf- bis sechzehnstündiger Bestrahlung pro 24 Stunden während des zweiten und dritten Schritts eingelegt wird, und während dieser Photoperiode die Bestrahlung der Keimlinge durch Lichtquellen, deren Verhältnis zwischen der im roten Bereich und im dunkelroten Bereich ausgestrahlten Energie größer oder gleich vier ist;
   (b) eine Phase der Induktion der Zwiebelbildung, umfassend einen Vermehrungszyklus, in dem eine Photoperiode von mindestens vierzehnstündiger Bestrahlung auf 24 Stunden während des zweiten und dritten Schritts eingelegt wird, und, während dieser Photoperiode, die Bestrahlung der Keimlinge durch mindestens eine Lichtquelle, deren Verhältnis rot/dunkelrot kleiner oder gleich vier ist, gewährleistet wird.

2. Verfahren nach Anspruch 1, dadurch **gekennzeichnet,** daß während der Phase der Induktion der Zwiebelbildung das Verhältnis rot/dunkelrot der Lichtquelle, die die Bestrahlung der Keimlinge gewährleistet, kleiner oder gleich 2 ist.

3. Verfahren nach einem der Ansprüche 1 oder 2, dadurch **gekennzeichnet,** daß während der Phase der Induktion der Zwiebelbildung eine Photoperiode, umfassend eine Bestrahlung zwischen 16 und 18 Stunden, während des zweiten und dritten Schritts des Vermehrungszyklus eingelegt wird.

4. Verfahren nach einem der Ansprüche 1 bis 3, dadurch **gekennzeichnet,** daß der erste Schritt jedes Vermehrungszyklus in Gegenwart mindestens eines Wuchsstoffes durchgeführt wird.

5. Verfahren nach einem der Ansprüche 1 bis 4, dadurch **gekennzeichnet,** daß während der Phase dar Verhinderung der Zwiebelbildung der zweite und der dritte Schritt des bzw. der Vermehrungszyklus/-zyklen in einem Milieu ohne Wachstumsfaktoren durchgeführt werden.

6. Verfahren nach Anspruch 5, dadurch **gekennzeichnet,** daß das Milieu mindestens 20 meq $NH_4^+$, mindestens 20 meq $NO_3^-$, mindestens 3 meq $PO_4^{3-}$ und zwischen 30 g/l und 60 g/l Zucker enthält.

7. Verfahren nach einem der Ansprüche 1 bis 6, dadurch **gekennzeichnet,** daß während der Phase der Verhinderung der Zwiebelbildung der zweite und der dritte Schritt des bzw. der Vermehrungszyklus/-zyklen bei einer Tagestemperatur zwischen +20 und +30°C und um mehr als +1°C über der Nachttemperatur durchgeführt werden.

8. Verfahren noch einem der Ansprüche 1 bis 7, dadurch **gekennzeichnet,** daß während der Phase der Induktion der Zwiebelbildung der zweite und der dritte Schritt des Vermehrungszyklus in einem Milieu, umfassend zwischen 30 g/l und 80 g/l Zucker, durchgeführt werden.

9. Verfahren nach einem der Ansprüche 1 bis 8, dadurch **gekennzeichnet,** daß während der Phase der Induktion der Zwiebelbildung der zweite und der dritte Schritt des Vermehrungszyklus in einem Milieu, umfassend zwischen 0 und 5 mg/l Abscisinsäure und/oder zwischen 0 und 1000 ppm Ethylen, durchgeführt werden.

10. Verfahren nach einem der Ansprüche 1 bis 9, dadurch **gekennzeichnet,** daß während der Phase der Induktion der Zwiebelbildung der zweite und der dritte Schritt des Vermehrungszyklus bei einer Tagestemperatur zwischen +20 und +35°C und einer Nachttemperatur zwischen +15 und +30°C durchgeführt werden.

11. Verfahren nach Anspruch 1, dadurch **gekennzeichnet,** daß es nur die Phase der Verhinderung der Zwiebelbildung umfaßt.

12. Verfahren nach Anspruch 1, dadurch **gekennzeichnet,** daß es nur die Phase der Induktion der Zwiebelbildung umfaßt.

FIGURE 1

Figure 1. - Cycle de multiplication illimitée de l'oignon (Allium cepa L.) in vitro